# EUROPÄISCHE PATENTANMELDUNG

(11) **EP 1 312 328 A2**
(43) Veröffentlichungstag der Anmeldung: **21.05.2003**
(21) Anmeldenummer: 02024353.1
(22) Anmeldetag: 02.11.2002
(51) Int. Cl.: A61F 13/537, A61F 13/539, A61F 13/15

(54) **Verbundmaterial für das Management von Körperflüssigkeiten**

(30) Priorität: 13.11.2001 DE 10155482
(71) Anmelder: Sandler AG, 95126 Schwarzenbach/Saale (DE)
(72) Erfinder:

(57) **Zusammenfassung**

Die vorliegende Erfindung handelt von einem Verbundmaterial (4) für das Management von Körperflüssigkeiten aus mindestens zwei fibrösen Materiallagen, bestehend aus einer klebstofffreien Verbindung aus einer Flüssigkeitsspeicherschicht (3) mit einer Stapelfasern (6) enthaltenden Flüssigkeitsverteilerschicht (2), wobei die Verbindung durch die am Übergang der Flüssigkeitsverteilerschicht (2) zur Flüssigkeitsspeicherschicht (3) befindliche Verankerungspunkte (1) erfolgt, die Verankerungspunkte (1) einerseits aus von der Flüssigkeitsspeicherschicht (3) emporragenden, multifilen Fasersträngen ( 5 ) und andererseits aus von der Flüssigkeitsverteilerschicht (2) abstehenden Stapelfasern (6) gebildet sind und die Faserstränge (5) und die Stapelfasern (6) form- und kraftschlüssig miteinander verbunden sind.

## Beschreibung

Die Entwicklung im Bereich der Hygieneartikel, namentlich Windeln, Slipeinlagen, aber auch bei Produkten für den Inkontinenzbereich, macht es erforderlich, für eine wiederholt rasche Flüssigkeitsaufnahme, -weiterleitung und -speicherung genau auf diesen Einsatzzweck abgestimmte Flüssigkeitsmanagementlagen einzusetzen.

Handelsübliche Hygieneartikel haben dabei den folgenden, schematischen Aufbau:
- dem Benutzer zugewandte Hüll-Lage aus Vlies und/oder Folie
- Flüssigkeitsverteilerschicht aus beispielsweise einem kardierten Vlies
- Flüssigkeitsspeicherschicht aus beispielsweise einem schmelzgeblasenen Vlies
- dem Benutzer abgewandte Wäscheschutzschicht aus Vlies oder Folie

Die Funktion der Flüssigkeitsverteilerschicht, ist dabei das rasche Aufnehmen von ausgeschiedenen Körperflüssigkeiten, sowie die gezielte Verteilung und Weiterleitung an die nachgeschaltete Saug-/ Speicherschicht.

Erreicht wird diese Funktion häufig durch die Kombination von offenen Strukturen im Bereich der Flüssigkeitsverteilerschicht aus beispielsweise kardiertem, grobtitrigen Faservlies mit guter Beständigkeit gegenüber Kompression und einer nachgeschalteten Flüssigkeitsspeicherschicht aus zum Beispiel schmelzgeblasenem Vliesstoff.

In dieser Kombination aus beispielsweise kardiertem und schmelzgeblasenem Vliesstoff müssen beide Lagen dergestalt miteinander verbunden sein, dass zum einen die Verarbeitungssicherheit gewährleistet, und, zum anderen sich diese positv auf die eigentliche Funktion, die Flüssigkeitsaufnahme und deren Weiterleitung und Speicherung, auszuwirkt.

Zwar gibt es auch nach dem Stand der Technik verschiedene Methoden, einen verarbeitungssicheren, verschiebegesicherten Verbund zu erreichen, jedoch haben sich daraus weitere inakzeptable Nachteile des Endproduktes ergeben.

So offenbart die US 5591149 eine mittels Druck und Hitze in einem Kalander erzeugte Verbindung. Diese Art der Verbindung bedient sich der speziellen Ausgestaltung des Kalanderprints, hat aber entscheidende Nachteile bezüglich der Materialauswahl und der Dicke des Aufbaus.

Ausgangsmaterialien mit stark verschiedenen Schmelzpunkten, wie beispielsweise Polyetheylen und Polyester, lassen sich nach diesem Verfahren nur schlecht verbinden. Desweiteren wird während des Verbindens auch die Dicke des Verbunds stark reduziert, sodaß ein nicht unerheblicher Teil der Grobstruktur in der Flüssigkeitsaufnahmezone inaktiviert wird, und sich letztendlich die Funktion verschlechtert.

Die Lehre der DE 199 277 85 zeigt ein mechanisches Verstricken von Fasern eines schmelzgeblasenen Vliesstoffes mit der offenen Struktur eines kardierten Vlieses.

Dies wird durch kurze schmelzgeblasene Fasern erreicht, welche sich in die offene Struktur des kardierten Vlieses einarbeiten. Da es sich hierbei nur um ein mechanisches Verstricken handelt, ist die Lagenhaftung entsprechend gering. Darüberhinaus zeigte sich, dass die Flüssigkeitstransportwirkung zwischen diesen Schichten, aufgrund der kurzen Fasern, nur unzureichend ist.

Ein mehrschichtiger Lagenaufbau, bei welchem der Verbund mittels Klebstoff erzielt wird, bringt zwar ausgezeichnete Lagenhaftungen, der Kleber wirkt jedoch als Trennschicht, somit ist die Flüssigkeitsverteilwirkung zu gering.

Der Erfindung lag daher die Aufgabe zugrunde, unter Vermeidung der vorgenannten Nachteile ein mehrschichtiges Verbundmaterial bereitzustellen, welches eine unbeschädigte grobstrukturierte Flüssigkeitsverteilerschicht und eine unbeschädigte feinstrukturierte Flüssigkeitsspeicherschicht hat, keine verteilungshemmende Grenzschicht aufweist und dennoch eine optimale Lagenhaftung hat.

Die Aufgabe wurde gemäß den Merkmalen des Anspruches 1 gelöst, vorteilhafte Ausgestaltungen sind in den Unteransprüchen 2 bis 16 genannt.

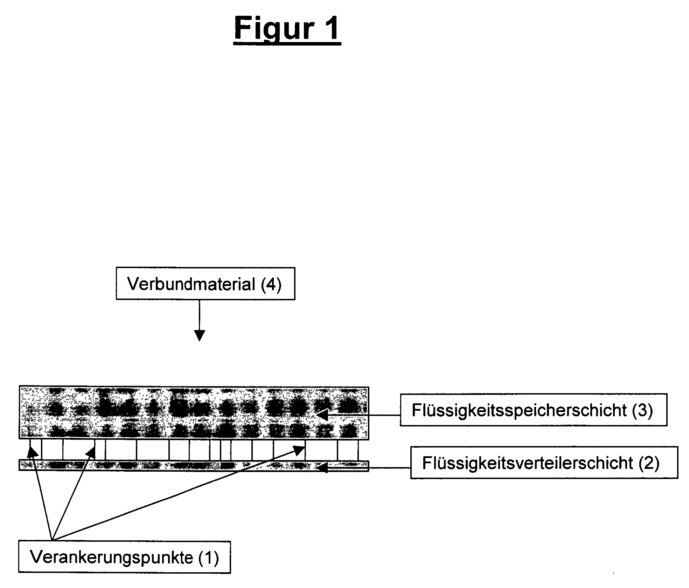

Figur 1 zeigt das erfindungsgemäß ausgebildete Verbundmaterial (4), bestehend aus einer Flüssigkeitsverteilerschicht (2), welche beispielsweise aus einem Stapelfasern (6) enthaltenden kardierten Vlies besteht, und der Flüssigkeitsspeicherschicht (3), welche beispielsweise aus einem schmelzgeblasene Fasern (8) und multifile Faserstränge (5) enthaltenden Vlies besteht.

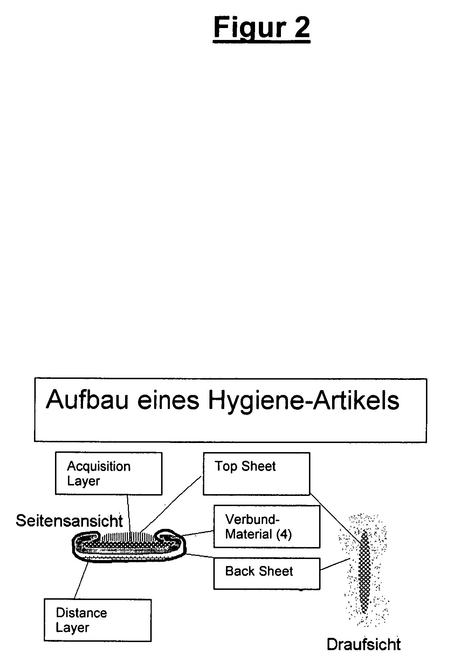

In Figur 2 wird die Verwendung des erfindungsgemäßen Verbundmaterials (4) anhand einer Querschnittszeichnung gezeigt.

Die Verbindung der Flüssigkeitsverteilerschicht (2) und der Flüssigkeitsspeicherschicht (3) wird während der Herstellung der Flüssigkeitsspeicherschicht (3) erzielt.

Dabei wird ein Basisvlies, welches im späteren Verbund die Flüssigkeitsverteilerschicht (2) bildet, als Trägermaterial eingesetzt.

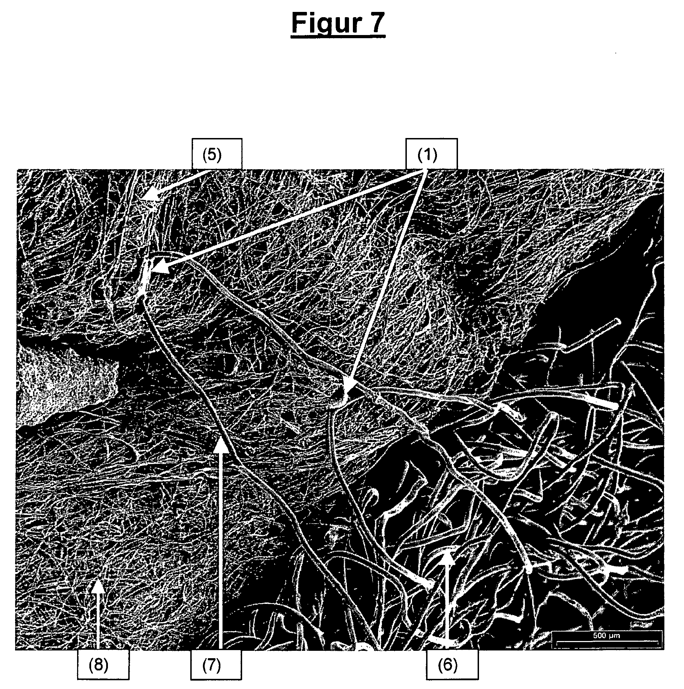

Die Bildung der Flüssigkeitsspeicherschicht (3) erfolgt nach dem Schmelzblasverfahren, wobei die Verfahrenseinstellungen gemäß den Angaben von Tabelle 1 gewählt werden. Das Trägermaterial wird dabei über die Kollektortrommel geführt, sodass sich die schmelzgeblasenen Fasern (8 in Figur 7) der Flüssigkeitsspeicherschicht (3) auf dem Trägermaterial sammeln und sich mit ihm erfindungsgemäß verbinden.

Dabei haben sich offenstrukturierte Stapelfaservliesstoffe als vorteilhaft erwiesen. Besonders günstig ist eine geringe Verfestigungsfläche im Bereich von 5 bis 15% bei thermisch kalanderverfestigten Vliesstoffen. Alternativ können aber auch hydrodynamisch verfestigte Stapelfaservliesstoffe oder thermisch, heißluftverfestigte Stapelfaservliesstoffe verwendet werden.

Die Verfahrenseinstellung an der Schmelzblaseinheit ist dabei so zu wählen, dass sich neben einzelnen Mikrofasern auch Zusammenballungen dieser, sogenannte multifile Faserstränge, ausbilden. Dabei werden durch die Wahl der Luftverhältnisse an der Düsenspitze der Schmelzblaseinheit Turbulenzen erzeugt, welche Zusammenballungen von gerade erzeugten schmelzgeblasenen Fasern (8 in Figur 7) zur Folge haben. Eine geeignete Methode zur Herstellung derartiger Vliesstoffe ist in DE 199 53 717 A1 offenbart.

Diese multifilen, schmelzgeblasenen Faserstränge (5 in Figur 7) haben, aufgrund der Zusammenballung mehrerer Einzelfasern zu einem Konglomerat, eine höhere Energiedichte als die ebenfalls nach der oben genannten Methode erhaltenen Fasern (8 in Figur 7). Dies bedingt, dass diese Faserstränge (5 in Figur 7) beim Auftreffen auf das vorgelegte Basisvlies noch weitgehend schmelzflüssig sind und daher aus dem Basisvlies herausstehende Stapelfasern (6 in Figur 7) regelrecht umfliessen.

Nach dem Erkalten entstehen kraft- und formschlüssige Verankerungspunkte (1) der multifilen Faserstränge (5 in Figur 7) des schmelzgeblasenen Vliesstoffes mit den Stapelfasern (6 in Figur 7) des Basisvliesstoffes.

Die kraftschlüssige Verbindung der multifilen Faserstränge (5 in Figur 7) mit den Stapelfasern (6 in Figur 7) an den Verankerungspunkten (1), bewirkt einerseits die mechanisch stabile Verbindung der Flüssigkeitsverteilerschicht (2) mit der Flüssigkeitsspeicherschicht (3), andererseits ermöglicht es auch die grenzschichtfreie Flüssigkeitsweiterleitung von der Flüssigkeitsverteilerschicht (2) zur Flüssigkeitsspeicherschicht (3) in Form von somit gebildeten Flüssigkeitsleitsträngen (7 in Figur 7).

Die Tatsache, dass diese Flüssigkeitsleitstränge (7 in Figur 7) sowohl in der Flüssigkeitsverteilerschicht (2) als auch in der Flüssigkeitsspeicherschicht (3) körperlich, dreidimensional verankert sind, stellt eine grenzschichtenfreie Verbindung der beiden Flüssigkeitsverteilerschicht (2) und Flüssigkeitspeicherschicht (3) dar, wobei die Flüssigkeitsleittränge (7 in Figur 7) eine Dochtwirkung aufgrund ihrer Kapillarität entwickeln.

Figur 7 zeigt eine REM-Aufnahme des Verbundmaterials (4) im Querschnitt, wobei die Ausbildung der Verankerungspunkte (1) zwischen den zwei Flüssigkeitsverteilerschicht (2) Flüssigkeitsspeicherschicht (3) optisch deutlich erkennbar ist.

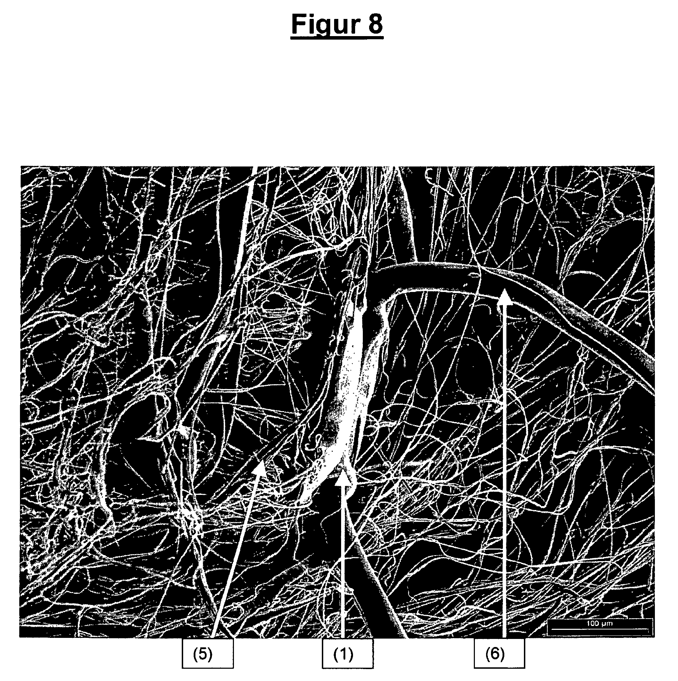

Figur 8 zeigt eine REM-Aufnahme eines Verankerungspunktes (1) von einem in der Flüssigkeitsspeicherschicht (3) enthaltenem multifilen schmelzgeblasenen Faserstrang (5) mit aus der Flüssigkeitsverteilerschicht (2) abstehenden Stapelfasern (6).

Eine geeignete Verfahrenseinstellung zur Erlangung eines erfindungsgemäßen Verbundmaterials (4) wird in der nachstehenden Tabelle 1 gezeigt.

**Tabelle 1:**

| Verfahrensparameter für Ausführungsbeispiel | |
|---|---|
| Volumenstrom V1 [ft³/min] | 280 |
| Volumenstrom V2 [ft³/min] | 350 |
| Temperatur Düsenspitze [°C] | 290 |
| Granulat | 100% Polypropylen HH 442H Basell |
| Quotient V1/V2 | 0,8 |
| Austrittswinkel α | ca. 47° |
| Ablagemedium | Kollektortrommel |
| Position des Ablagemediums | Zenit unter Düsenöffnung |
| Abzugsrichtung | in Öffnungsrichtung des Abzugswinkels α |
| Faserkräuselung | Ja |
| Faserdurchmesser des schmelzgeblasenen Faservlieses von-bis | 2 bis ca. 47 µm |
| Flächenmasse des schmelzgeblasenen Faservlieses | 80 g/m² |
| Basisvlies | Kardiertes, thermisch-kalanderverfestigtes Stapelfaservlies aus 100% PES-Faser 6.7 dtex/40mm, 25 g/qm |

Die nachfolgende Tabelle 2 beschreibt die verschiedenen Eigenschaften des Verbundmaterials im Vergleich zum Stand der Technik.

**Tabelle 2:**

| Vergleich verschiedener Eigenschaften des erfindungsgemäßen Verbundmaterials zum Stand der Technik | | | | | | | | |
|---|---|---|---|---|---|---|---|---|
| Produkt | Gewicht (g/qm) | Dicke (mm) | Lagenhaftung (N/5cm) | Ansaugzeit (sec/20 ml) | Rücknässung (g/20ml) | Flüssigkeitsverteilwirkung (Ausbreitungsdurchmesser in mm) | | |
| | | | | | | nach 60 sec | nach 120 sec | nach 240 sec |
| Klebstoffverbund | 110 | 1,32 | 1,35 | 36,4 | 12,20 | 20 | 42 | 58 |
| Kalanderverbund | 104 | 0,86 | 1,21 | 26,5 | 8,61 | 36 | 52 | 64 |
| Overblow nach dem Stand der Technik | 100 | 1,55 | 0,17 | 17,4 | 3,87 | 57 | 75 | 94 |
| Erfindungsgemäßes Verbundmaterial (4) | 105 | 1,76 | 0,80 | 14,8 | 2,57 | 56 | 95 | 119 |

Wobei die Zusammensetzung der verwendeten Materialien sich wie folgt darstellt:

Klebstoffverbund: Flüssigkeitsaufnahmeschicht aus kardiertem Vlies (25 g/qm) aus 100% PES-Faser, 6.7 dtex, mittels 3 g/qm Hotmeltklebstoff mit einer Flüssigkeitsspeicherschicht aus einem PP-Meltblownvliesstoff (80 g/qm) verbunden.

Kalanderverbund: Flüssigkeitsaufnahmeschicht aus kardiertem Vlies (25 g/qm) aus 100% PP-Faser, 6.7 dtex, mit einer Flüssigkeitsspeicherschicht aus einem PP-Meltblownvliesstoff (80 g/qm) thermisch verbunden.

Overblow nach dem Stand der Technik: Flüssigkeitsaufnahmeschicht aus kardiertem Vlies (25 g/qm) aus 100% PES-Faser, 6.7 dtex, mit einer Flüssigkeitsspeicherschicht aus einem PP-Meltblownvliesstoff (75 g/qm) verbunden.

Erfindungsgemäßes Verbundmaterial (4): Flüssigkeitsverteilerschicht (2) aus kardiertem Vlies (25 g/qm) aus 100% PES-Stapelfasernfaser (6), 6.7 dtex, mit einer Flüssigkeitsspeicherschicht (3) aus einem PP-Meltblownvliesstoff (80 g/qm) verbunden.

Als Testmethoden kamen dabei folgende zur Anwendung:
- Gewicht:: gemäß EDANA 40.3 - 99
- Dicke:: gemäß EDANA 30.5 - 99, Messdruck 0,02 kPa
- Lagenhaftung:: gemäß DIN 53539
- Ansaugzeit:: gemäß EDANA 150.4 - 99, mit 20 ml Testflüssigkeit
- Rücknässung:: gemäß EDANA 151.2 - 99, mit 20 ml Testflüssigkeit

Flüssigkeitsverteilwirkung: Ausbreitung einer Flüssigkeitsmenge von 20 ml 0,9% NaCI-Lösung innerhalb der Flüssigkeitsspeicherschicht (3), Angabe des Durchmessers der flüssigkeitsdurchdrungenen Fläche nach verschiedenen Einwirkzeiten in mm.

Aus der Tabelle 2 ist ersichtlich, dass die erfindungsgemäß hergestellten Verbundmaterialien (4) sowohl von der Aufnahmezeit als auch von ihrer Verteilwirkung her, den herkömmlichen, dem Stand der Technik entsprechenden Flüssigkeitstransportlagen weit überlegen sind.

So zeigt sich, dass bei vergleichbaren Flächengewichten die Ansaugzeit des erfindungsgemäßen Verbundmaterials (4) bis zu 50 Prozent geringer liegt als bei konventionellen Produkten.

Dies trifft auch für die Rücknässung zu. Hier zeigt sich der negative Einfluss von Barriere-Effekten bei z.B klebstoff-basierten Verbunden, welche bis zu sechsmal höhere Rücknässung haben als erfindungsgemäß hergestellte Verbundmaterialien (4).

Auch ist die Flüssigkeitverteilwirkung, ein Maß für die Effizienz des Fluidmanagements, der des Stands der Technik deutlich überlegen. Gleiche Flüssigkeitsmengen werden auf eine bis zu 5-fach größere Fläche der Flüssigkeitsspeicherschicht (3) verteilt, als dies bei Materialien nach dem Stand der Technik der Fall ist.

Hier zeigt sich das positive Zusammenwirken von grenzschichtfreiem Verbund und den erfindungsgemäß erzeugten Flüssigkeitsleitsträngen (7), sodaß ein ungehemmter Fluidtransport von der Flüssigkeitsverteilerschicht (2) zur Flüssigkeitsspeicherschicht (3), sowie innerhalb der Flüssigkeitsspeicherschicht (3) stattfinden kann.

Ein Rücktransport von bereits gespeicherter Flüssigkeit, auch bezeichnet als Rücknässung, wird aufgrund des von der Flüssigkeitverteilerschicht (2) hin zur Flüssigkeitsspeicherschicht (3) abnehmenden Fasertiters und der daraus resultierenden, zunehmenden Kapillarwirkung vermieden.

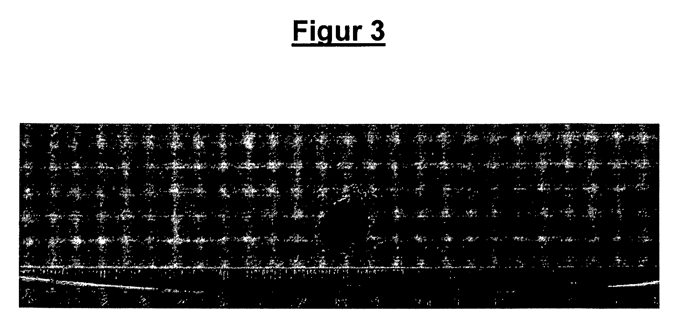

Die Flüssigkeitsverteilwirkung über die Zeit verdeutlichen auch die Figuren 3 - 6. Hier kann die Ausbreitung von 20ml, mit Lebensmittelfarbstoff eingefärbter 0.9% NaCI-Lösung beobachtet werden.

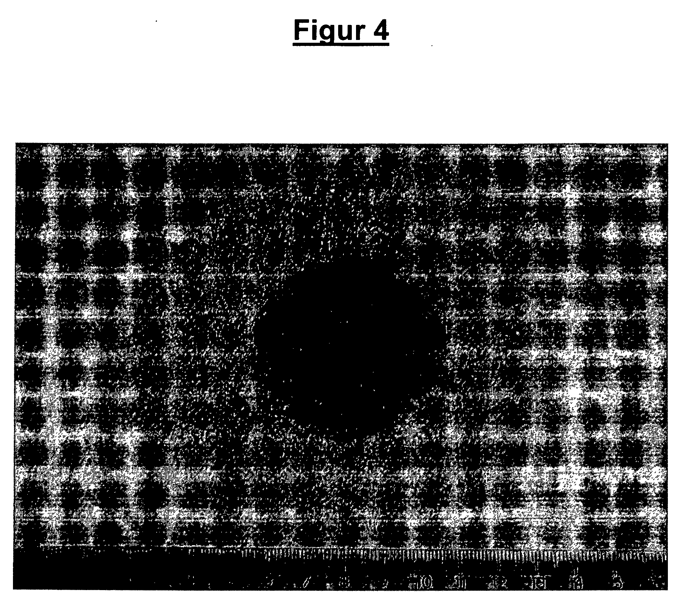

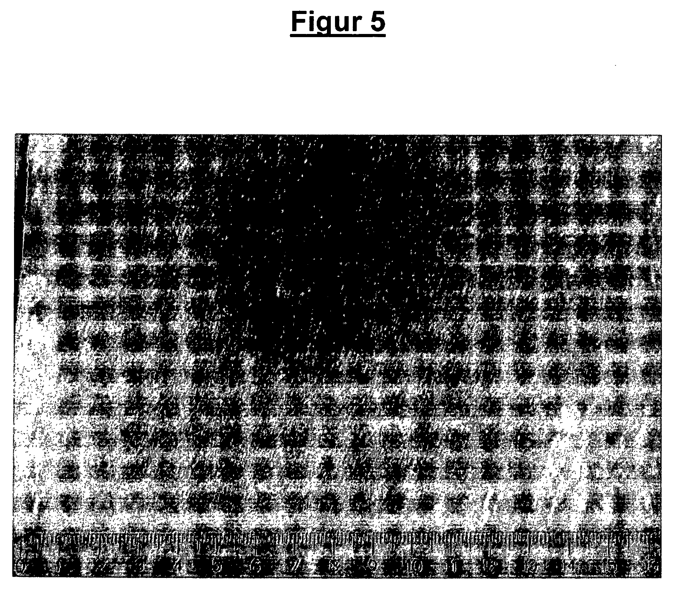

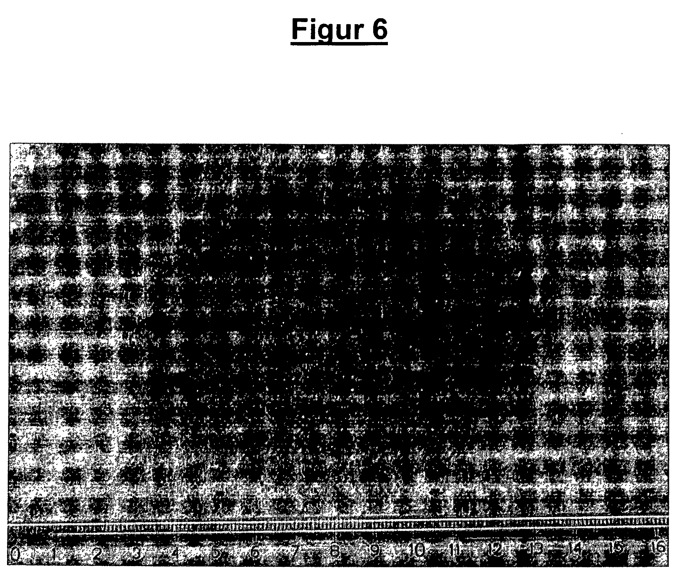

In Figur 3 wird die Flüssigkeit zunächst in der Flüssigkeitsverteilerschicht (2) angenommen und in den Figuren 4 und 5 an die Flüssigkeitsspeicherschicht (3) weitergeleitet . Durch die erfindungsgemäß ausgebildeten Flüssigkeitsleitstränge (7) erfolgt der Flüssigkeitstransport jedoch nicht nur in vertikaler Richtung, sondern zeitgleich auch entlang der Flüssigkeitsleitstränge (flGUR7) in quasi horizontaler Richtung, sodaß eine effizientere Auslastung der Flüssigkeitsspeicherschicht (3), bis hin zur vollständigen Entlastung der Flüssigkeitsverteilerschicht (2), gegeben ist (vergleiche hierzu Figur 6).

Bei Anwendung des erfindungsgemäßen Verbundmaterials (4) ist es auch unerheblich, welchen Schmelzpunktunterschied die verwendeten Basismaterialien aufweisen, da letztendlich nur das Polymer zur Herstellung des schmelzgeblasenen Vliesstoffes zum Schmelzen gebracht werden muss.

Erfindungsgemäß hergestellte Verbundmaterialien (4) eignen sich daher als universell einsetzbare Fluidmanagement-Komponenten in den verschiedensten Hygieneprodukten, sie sind daher im Damenhygienebereich, Windelbereich, aber auch im Inkontinenzbereich einsetzbar.

### Bezugszeichenliste zur E 17/01 "Verbundmaterial für das Management von Körperflüssigkeiten"

1 - Verankerungspunkt
2 - Flüssigkeitsverteilerschicht
3 - Flüssigkeitsspeicherschicht
4 - Verbundmaterial
5 - Multifile Faserstränge
6 - Stapelfasern
7 - Flüssigkeitsleitstrang
8 - Fasern

## Patentansprüche

1. Verbundmaterial (4) für das Management von Körperflüssigkeiten aus mindestens zwei fibrösen Materiallagen, bestehend aus einer klebstofffreien Verbindung aus einer Flüssigkeitsspeicherschicht (3) mit einer Stapelfasern (6) enthaltenden Flüssigkeitsverteilerschicht (2),
**dadurch gekennzeichnet,**
- **dass** die Verbindung durch die am Übergang der Flüssigkeitsverteilerschicht (2) zur Flüssigkeitsspeicherschicht (3) befindliche Verankerungspunkte (1) erfolgt,
- **dass** die Verankerungspunkte (1) einerseits aus von der Flüssigkeitsspeicherschicht (3) emporragenden, multifilen Fasersträngen ( 5 ) und andererseits aus von der Flüssigkeitsverteilerschicht (2) abstehenden Stapelfasern (6) gebildet sind und
- **dass** die Faserstränge (5) und die Stapelfasern (6) form- und kraftschlüssig miteinander verbunden sind.

2. Verbundmaterial (4) für das Management von Körperflüssigkeiten nach Anspruch 1,
**dadurch gekennzeichnet,**
**dass** die mit den Stapelfasern (6) verbundenen Faserstränge (5) Flüssigkeitsleitstränge (7) zwischen der Flüssigkeitsverteilerschicht (2) und der Flüssigkeitsspeicherschicht (3) bilden.

3. Verbundmaterial (4) für das Management von Körperflüssigkeiten nach Anspruch 1,
**dadurch gekennzeichnet,**
**dass** der Flächenanteil aller Verankerungspunkte (1) zwischen Flüssigkeitsspeicherschicht (3) und Flüssigkeitsverteilerschicht (2) höchstens 10 Prozent der Gesamtfläche beträgt.

4. Verbundmaterial (4) für das Management von Körperflüssigkeiten nach Anspruch 1,
**dadurch gekennzeichnet,**
**dass** die Flüssigkeitsspeicherschicht (3) und die Flüssigkeitsverteilerschicht (2) aus thermoplastischen Polymeren bestehen.

5. Verbundmaterial (4) für das Management von Körperflüssigkeiten nach Anspruch 4,
**dadurch gekennzeichnet,**
**dass** diese thermoplastischen Polymere aus der Gruppe der Polyolefine gewählt sind.

6. Verbundmaterial (4) für das Management von Körperflüssigkeiten nach Anspruch 4,
**dadurch gekennzeichnet,**
**dass** diese thermoplastischen Polymere aus der Gruppe der Polyethylenterephthalate gewählt sind.

7. Verbundmaterial (4) für das Management von Körperflüssigkeiten nach Anspruch 4,
**dadurch gekennzeichnet,**
**dass** diese thermoplastischen Polymere aus der Gruppe der Polyamide gewählt sind.

8. Verbundmaterial (4) für das Management von Körperflüssigkeiten nach Anspruch 1,
**dadurch gekennzeichnet,**
**dass** die in der Flüssigkeitsverteilerschicht (2) enthaltenen Stapelfasern (6) eine Feinheit von mindestens 3 bis höchstens 16 dtex besitzen.

9. Verbundmaterial (4) für das Management von Körperflüssigkeiten nach Anspruch 1,
**dadurch gekennzeichnet,**
**dass** die die Flüssigkeitsspeicherschicht (3) bildenden Fasern (8) und Filamentstränge (5) einen Feinheitsbereich von mindestens 0,3 bis höchstens 20,0 dtex besitzen.

10. Verbundmaterial (4) für das Management von Körperflüssigkeiten nach Anspruch 1,
**dadurch gekennzeichnet,**
**dass** das Verbundmaterial (4) eine Dicke von mindestens 0,1 bis höchstens 4,5 mm aufweist.

11. Verbundmaterial (4) für das Management von Körperflüssigkeiten nach Anspruch 1,
**dadurch gekennzeichnet,**
**dass** das Verbundmaterial (4) ein Flächengewicht von mindestens 20 bis höchstens 500 g/m² besitzt.

12. Verbundmaterial (4) für das Management von Körperflüssigkeiten nach Anspruch 1,
**dadurch gekennzeichnet,**
**dass** die Flüssigkeitsspeicherschicht (3) und/oder die Flüssigkeitsverteilerschicht (2) farbig ausgestaltet sind/ist.

13. Verbundmaterial (4) für das Management von Körperflüssigkeiten nach Anspruch 1,
**dadurch gekennzeichnet,**
**dass** die Faserstränge (5) der Flüssigkeitsspeicherschicht (3) und/oder die Stapelfasern (6) der Flüssigkeitsverteilerschicht (2) an ihren Oberflächen mit einer hydrophilen Ausrüstung versehen sind.

14. Verbundmaterial (4) für das Management von Körperflüssigkeiten nach Anspruch 1,
**dadurch gekennzeichnet,**
**dass** die Faserstränge (5) der Flüssigkeitsspeicherschicht (3) und/oder die Stapelfasern (6) der Flüssigkeitsverteilerschicht (2) an ihren Oberflächen mit einer antibakteriell wirksamen Ausrüstung versehen sind.

15. Verbundmaterial (4) für das Management von Körperflüssigkeiten nach Anspruch 1,
**dadurch gekennzeichnet,**
**dass** die Stapelfasern (6) enthaltende Flüssigkeitsverteilerschicht (2) ein hydrodynamisch verfestigter Vliesstoff ist.

16. Verbundmaterial (4) für das Management von Körperflüssigkeiten nach Anspruch 1,
**dadurch gekennzeichnet,**
**dass** die Stapelfasern (6) enthaltende Flüssigkeitsverteilerschicht (2) ein thermisch verfestigter Vliesstoff ist.
